# EUROPEAN PATENT SPECIFICATION

(11) **EP 2 775 945 B1**
(45) Date of publication and mention of the grant of the patent: **08.05.2019**
(21) Application number: 12847102.6
(22) Date of filing: 13.11.2012
(51) Int. Cl.: A61B 17/3207, A61B 17/22, A61B 1/04, A61M 25/01

(54) **OCCLUSION-CROSSING DEVICES, ATHERECTOMY DEVICES, AND IMAGING**
OKKLUSIONSDURCHQUERUNGSVORRICHTUNGEN, ATHEREKTOMIEVORRICHTUNGEN UND BILDGEBUNG
DISPOSITIF DE TRAVERSE D'OCCLUSION, DISPOSITIFS D'ATHÉRECTOMIE, ET IMAGERIE

(30) Priority: 11.11.2011 US 201161559013 P; 28.03.2012 US 201213433049
(43) Date of publication of application: 17.09.2014
(73) Proprietor: Avinger, Inc., Redwood City, CA 94063 (US)
(72) Inventor: SPENCER, Maegan K., Redwood City, CA 94063 (US); SIMPSON, John B., Redwood City, CA 94063 (US); DAVIES, Stephen C., Redwood City, CA 94063 (US)
(74) Representative: Potter Clarkson
(86) International application number: PCT/US2012/064848
(87) International publication number: WO 2013/071299

(56) References cited:
- EP-A2- 0 347 098
- US-A- 4 926 858
- US-A- 5 041 082
- US-A1- 2003 018 346
- US-A1- 2003 125 757
- US-A1- 2005 187 571
- US-A1- 2005 187 571
- US-A1- 2007 010 840
- US-A1- 2007 276 419
- US-A1- 2007 276 419

## Description

### CROSS REFERENCE TO RELATED APPLICATIONS

This application claims the benefit under 35 U.S. C. 119 of U.S. Provisional Patent Application No. 61/559,013, filed November 11, 2011, titled "ATHERECTOMY METHODS AND DEVICES." This application is also a continuation-in-part of U.S. Patent Application No. 13/433,049, filed March 28, 2012, titled "OCCLUSION-CROSSING DEVICES, IMAGING, AND ATHERECTOMY DEVICES," now Publication No. US-2012-0253186-A1.

### FIELD

The devices, methods and systems described herein are related to the treatment, identification, and removal of atheroma. In particular, described herein are systems, methods, devices and techniques for identifying distinguishing morphology in vessel images to direct or orient interventional devices.

### BACKGROUND

Coronary artery disease is the leading cause of death within the United States for men and women. It is characterized by a buildup of material (often fatty) in the internal lumen of the coronary arteries. It is also associated with the hardening of the arterial walls. The buildup of material commonly starts on one side of the vessel and grows across the open lumen. As such, the last point of patency often occurs at the boundary between the material deposit (disease) and the healthy vessel.

Atherectomy is the process of removing diseased tissue from a stenosed lumen so as to restore patency and blood flow. There currently exist a number of devices that facilitate atherectomy. However, the operation of such devices has a number of shortcomings. In some instances, the active element of the atherectomy device acts equally in all directions, requiring the device to reside in the center of the diseased portion to maintain optimum efficacy. In other instances, the active element is directional but as such needs some method of visualization to orient the active element with respect to the diseased tissue. In many instances, the method of visualization that is employed is angiography, which is only capable of giving a silhouette of the open lumen.

Further, minimally invasive techniques for treating coronary artery disease, such as atherectomy, typically involve the placement of a guidewire through the occlusion prior to performing the atherectomy. For example, a chronic total occlusion (CTO) device can be used to place a guidewire through the occlusion and ultimately cross through the occlusion. Unfortunately, placement of the guidewire, while critical for effective treatment, may be difficult. In particular, when placing a guidewire across an occlusion, it may be difficult to pass the guidewire through the occlusion while avoiding damage to the artery. For example, it is often difficult to prevent the guidewire from directing out of the lumen into the adventitia and surrounding tissues, potentially damaging the vessel and preventing effective treatment of the occlusion.

Moreover, minimally invasive surgical procedures to treat coronary artery disease depend on the precise positioning and manipulation of interventional devices. Guidance provided by high-resolution imaging can enable the characterization of tissue and lesion properties in vivo prior to treatment. As the majority of atherogenesis occurs in an eccentric fashion within the artery, therapeutic tools that have onboard imaging provide a distinct opportunity to selectively treat the diseased portion of a vessel. Even with on-board imaging techniques, however, it can be difficult to interpret the images so as to properly orient and steer the interventional devices as needed.

Accordingly, there is a need for a consistent and precise mechanism for steering or orienting occlusion-crossing, atherectomy, or other interventional devices. The invention described herein is based on the novel realization that a characteristic morphology (or morphological structure) may be visualized when (or after) passing a structure through the lumen of a vessel containing an atherectomy plaque mass (atheroma).

US 2007/276419 A1 discloses an atherectomy device having a distal end configured to dissect plaque from within a vessel, the device comprising an elongate catheter body, a troweled distal tip extending from the catheter body, the troweled distal tip having a curved outer surface configured to conform to an outer vessel, a rotatable cutter at least partially within the troweled distal tip and a drive cable within a lumen of the catheter body configured to actuate the cutter.

### SUMMARY OF THE DISCLOSURE

According to the present invention there is provided an atherectomy device according to claim 1. Preferred embodiments of the invention are described in the dependent claims. Also described herein are methods of forming and/or identifying characteristic morphologies within a vessel that indicate the presence, orientation and location of plaque masses within the vessel. Also described are devices to image, identify, and use this characteristic morphology (e.g., morphological structure) to orient a device, and/or remove or navigate the plaque in the peripheral or coronary vasculature.

Described herein is a method of identifying an atherectomy plaque mass in a vessel that includes applying circumferential radial force within the vessel to displace a rigid plaque mass and force the vessel wall to stretch away from the device; imaging the vessel to create an image; and identifying crescent-shaped structures associated with an atherectomy plaque in the image.

This and other methods can include one or more of the following features. The method can further include identifying the orientation of a plaque mass based on the directionality of the crescent-shaped structures. The method can further include identifying the position of a plaque mass relative to outer layered structures of a vessel wall based on the crescent-shaped structures. Imaging the vessel can include imaging the vessel with optical coherence tomography. The method can further include inserting a device into the vessel, and the device can apply the circumferential radial force. Imaging the vessel can include imaging with an imaging sensor attached to the device. The method can further include orienting the device within the vessel based on the crescent-shaped structures. Orienting the device can include pointing a directional cutter at a plaque mass identified based upon the crescent-shaped structure. Orienting the device can include directing the device based upon the relationship between markers in the image and the crescent-shaped structures. The method can further include rotating the imaging sensor to obtain the image.

Another method of identifying an atherectomy plaque mass within a vessel includes the steps of: applying circumferential radial force within the vessel to displace a rigid plaque mass and forcing the vessel wall to stretch away from the device; visualizing the vessel wall following the application of circumferential radial force; and identifying crescent-shaped structures. The crescent-shaped structures may be formed by the application of circumferential radial force from within the lumen of the vessel.

This and other methods may include one or more of the following features. The method may also include the step of identifying the orientation of a plaque mass based on the directionality of the crescent-shaped structures. In some variations, the method may also include the step of identifying the position of a plaque mass relative to the outer layered structures of a vessel based on the crescent-shaped structures. In some variations, the method may also include the step of orienting a device or therapeutic tool within the vessel based on the crescent-shaped structures.

Any of the methods described herein can be carried out by a controller. Thus, an imaging system can be configured to detect, label, and/or highlight the characteristic morphological structures and /or use them to automatically detect or suggest the location of an atheroma.

The atherectomy device includes a distal end configured to dissect plaque from within a vessel. The device includes an elongate catheter body, a troweled distal tip extending from the catheter body and a rotatable cutter. The troweled distal tip has a curved outer surface configured to conform to an outer vessel and a scooped inner edge configured to at least partially plane along the plaque. The rotatable cutter is at least partially within the troweled distal tip.

This and other embodiments can include one or more of the following features. The device can further include an OCT sensor near or on the rotatable cylindrical cutter and configured to image radially into the vessel. The device can further include an inner lumen opening through the rotatable cutter into which material cut by the device may be driven. The rotatable cutter can be partially covered by the curved outer surface and can be partially exposed proximate to the scooped inner edge. The device can be configured to self-orient within the vessel.

The device may include a rotatable drive shaft for rotating the OCT sensor. Other elements may also or alternatively be included. The distal tip is generally trowel or shovel-shaped in order to match the morphology of the plaque/wall interface revealed by the characteristic crescent shape described herein.

### BRIEF DESCRIPTION OF THE DRAWINGS

FIGS. 1-3 show examples of OCT images from within the lumen of a blood vessel, illustrating the crescent-shaped morphology described herein.
FIG. 4 illustrates one variation of an atherectomy device adapted as described herein.
FIGS. 5 and 6 illustrate an exemplary device with markers configured to display on an OCT image.
FIG. 7 illustrates one method of detecting crescent-shaped structures indicative of the boundaries of an atheroma as described herein.

### DETAILED DESCRIPTION

When crossing an occlusion of a blood vessel and imaging the vessel, such as with optical coherence tomography (OCT), an unexpected, yet predictable and characteristic morphology (or geometry) can be identified. The resulting characteristic morphology, described further herein, is formed at the boundary between the layered vessel wall structures and a plaque mass when crossing a chronic total occlusion (CTO) with a catheter or crossing device such as those described in: U.S. Patent Application No. 12/689,748, filed January 19, 2010, titled "GUIDEWIRE POSITIONING CATHETER," now Publication No. US-2010-0274270-A1; U.S. Patent Application No. 12/108,433, filed April 23, 2008, titled "CATHETER SYSTEM AND METHOD FOR BORING THROUGH BLOCKED VASCULAR PASSAGES," now Patent No. 8,062,316; U.S. Patent Application No. 12/829,277, filed July 1, 2010, titled "ATHERECTOMY CATHETER WITH LATERALLY-DISPLACEABLE TIP," now Publication No. US-2011-0004107-A1; U.S. Patent Application No. 12/829,267, filed July 1, 2010, titled "CATHETER-BASED OFF-AXIS OPTICAL COHERENCE TOMOGRAPHY IMAGING SYSTEM," now Publication No. US-2010-0021926-A1; U.S. Patent Application No. 12/790,703, filed May 28, 2010, titled "OPTICAL COHERENCE TOMOGRAPHY FOR BIOLOGICAL IMAGING," now Publication No. US-2010-0305452-A1; U.S. Patent Application No. 13/175,232, filed July 1, 2011, titled "ATHERECTOMY CATHETERS WITH LONGITUDINALLY DISPLACEABLE DRIVE SHAFTS," now Publication No. US-2012-0046679-A1; U.S. Patent Application No. 13/433,049, filed March 28, 2012, titled "OCCLUSION-CROSSING DEVICES, IMAGING, AND ATHERECTOMY DEVICES," now Publication No. US-2012-0253186-A1. The characteristic morphology may formed as a result of a dissection plane that is generated between a plaque mass and the lumen walls while traversing a vascular lesion, e.g., in peripheral and coronary CTO intervention.

As described herein, the resulting morphology is highly characteristic and can be used to obtain information with respect to the position of the disease within and with respect to the vessel. This information can also be utilized in real-time to orient the therapeutic portion of a device (e.g., atherectomy cutter or pre-shaped tip, etc.) toward disease and away from healthy tissue.

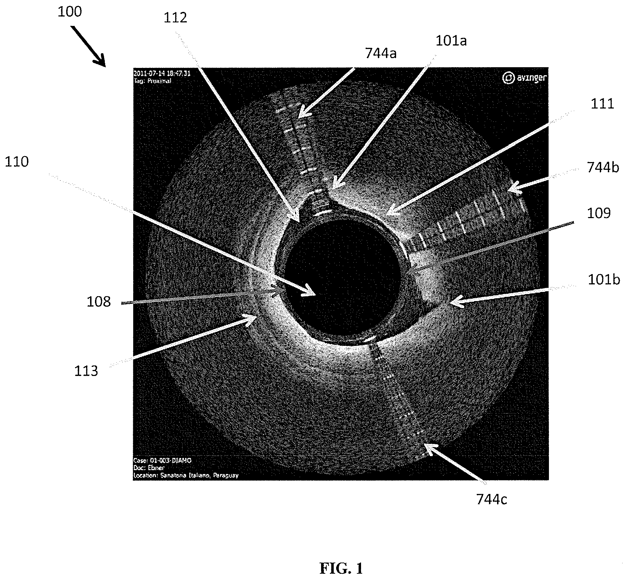

Using an imaging modality, such as optical coherence tomography (OCT), it is possible to visualize the shape or geometry that is created when advancing a device through a vascular lesion, such as a CTO. In some instances, the distinguishing shape or morphology that results when passing through a vascular lesion is a crescent-shaped feature (or a pair of such features) which may be formed around the circumference of the device, as shown in FIGS. 1-3.

In FIGS. 1-3, the images are gathered by an occlusion-crossing catheter having a rotatable distal tip which allows the tip to be driven past the occluded region. The images in FIGS. 1-3 are generated by rotating an OCT imaging sensor around the lumen of the vessel. The tip of the catheter may be rotated both (or either) clockwise or counterclockwise. The rate of rotation may be constant or variable, and may be between about 1 and about 5000 rpms. The OCT imaging sensor may be located on the catheter (e.g., at or near the distal end), and the sensor may be rotated to enable circumferential imaging of the lumen (by rotating an end portion of the catheter, for example). The catheter may be held in a relatively fixed position within the lumen of the vessel or it may be moved longitudinally through the lumen while obtaining the images.

For example, FIG. 1 shows an OCT image taken from a catheter equipped to take OCT images from within a lumen of a vessel that is at least partially occluded. In this example, a catheter (which may include the OCT imaging catheter) has been inserted past the occluded region shown. In FIG. 1, the tip of the catheter has already passed through this occluded region of the vessel, and OCT image 100 was taken of the vessel after passage of the tip.

The image 100 shown includes a black circle 110, which is representative of the device itself. Around the black circle 110 extends a dark portion 112. The dark portion 112 forms a semi-circular-like shape (or a "D" shape) with a rounded portion 108 on one side and a substantially linear portion 109 on the other side (note that the substantially linear portion is shorter than the rounded portion 108 and can be slightly bowed in the opposite direction). The rounded portion 108 and the substantially linear portion 109 meet in characteristic crescents or "crescent wings" 101a, 101b (in this image 100 at the 11 and 4 o'clock positions, respectively) to give a "cat ear" shaped profile. The crescent wings 101a, 101b point inwards (towards the substantially linear portion 109) to frame an amorphous structure 111 indicating a plaque mass in the vessel. In contrast, the rounded portion 108 lies against layered structures 113 of different contrast, indicating healthy tissue of the vessel (e.g., intima, adventitia, media).

Although the inventions described herein are not bound by any particular theory, the characteristic morphology of bracketing crescents ("cat ears") shown and described with respect to FIG. 1 is may be primarily due to the tendency for a device inserted within the vessel to track along the side of a plaque mass (between the plaque mass and the vessel wall) rather than through the plaque mass. As the device tracks along the side of the plaque mass, the wall of the vessel stays intact and stretches, thereby bowing out in a substantially circumferential manner along with the device (forming the rounded portion 108). In contrast, the plaque mass may undergo little, if any, stretching, thereby maintaining a substantially straight profile (forming the substantially linear portion 109). The dark portion 112 may be formed as blood is cleared by flushing through a lumen on the device or peripheral apparatus. The pointed portion of the crescent wings 101a, 101b may form as a result of the healthy tissue stretching around the cylindrically-shaped device, which is positioned between the plaque mass and the vessel outer wall (e.g., adventitia). Further, the black circle 110 is a feature that is added (e.g., by software) into the image to indicate the approximate shape and location of the device relative to the environment; the OCT sensor in this example does not image within the device itself.

Thus, the characteristic morphology described with respect to FIG. 1 may be mainly due to the tendency for a device placed within a clogged vessel to track along the side of a plaque mass. The morphology along this path may promote anisotropic stretching around the device. When circumferential radial forces are applied in this space, the rigid plaque mass is less likely to accommodate the introduced device, forcing the more compliant vessel wall to preferentially stretch away from the device. When visualized with onboard imaging, this morphology appears as crescent-shaped edges, with wings (crescent edges) that occur on each side of a plaque region. These wings (also referred to as crescents or cat ears) are typically bowed in one direction, forming a half-moon shape, as shown in FIG. 1. The directionality of these wings may be indicative of the position of a plaque mass relative to the outer layered structures of a vessel, e.g., the smaller side of the shape may be on the side of the plaque region. This shape is consistent with predications based on the relative compliance of vessel wall structures and disease.

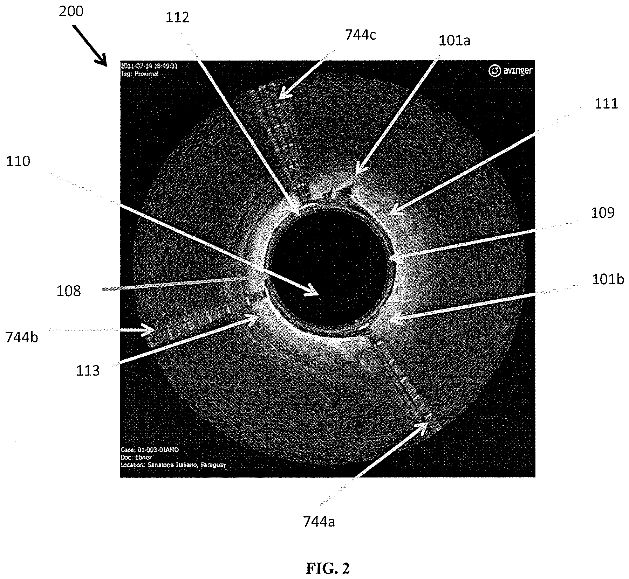

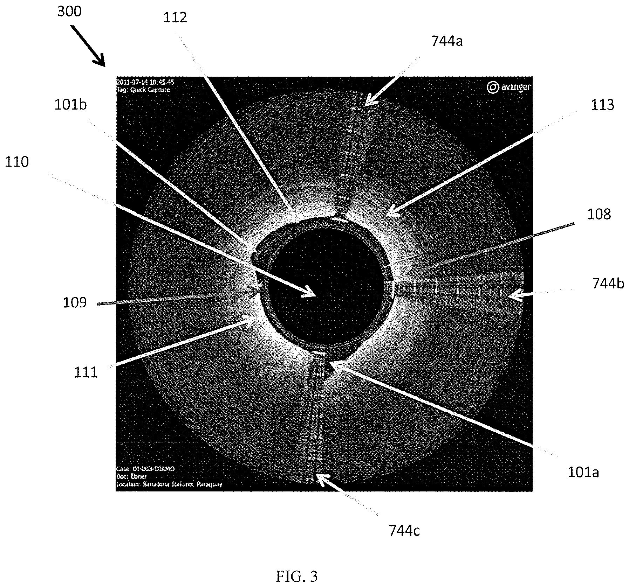

Additional representative OCT images 200, 300 are also shown in FIGS. 2 and 3. Each image 200, 300 includes the same or similar characteristic morphology as described with respect to FIG. 1. The images 100, 200, and 300 all show the morphology in a slightly different orientation (for example, the crescent wings 101a, 101b are in the 11 o'clock and 4'olock positions in image 100 and the crescent wings 101a, 101b in the 6 o'clock and 10 o'clock positions in the figure 300). The second crescent shape in FIG. 2 101b is partially hidden behind the reference structure 744a. This difference in orientation between the images is a result of the varying nature of disease within a vessel and is dependent on the path that a CTO-crossing device takes while traversing through a lesion. This orientation may actively change while traversing a device through a diseased segment of a vessel.

The characteristic morphology shown in FIGS. 1-3 can be used as a marker to provide a real-time roadmap during an interventional procedure in diseased vasculature to aid in device placement. That is, the crescent-shaped characteristic features, with a plaque mass on one side and layered wall structures on the other, can be used as a clear guide during interventional procedures in the peripheral or coronary vasculature.

Further, the tip of an atherectomy and/or imaging device (including, for example, the cutting element or occlusion-crossing element) can be repositioned toward a plaque mass using the crescent morphology as a guide, enabling a device to track along the true lumen of a CTO and avoid perforation of the vessel. A therapeutic tool may also be positioned using this feature to remove or modify a plaque mass and avoid the outer wall structures of a vessel, preventing vessel perforation.

In one embodiment, marker features (e.g., fiducial markers) on the device can assist in aligning the device in the desired orientation relative to the crescent morphology. The markers can be configured to obstruct imaging from the OCT sensor at least once per rotation of the rotatable tip. For example, the markers on the device can be a radiopaque material (e.g., a metal) that can be seen in high contrast during fluoroscopy or a material that reflects or absorbs optical beams from the OCT system (e.g., metal, dense polymer, carbon powder). As described in more detail below, the imaging system may also be configured to identify, mark, and/or highlight these characteristic crescent morphological shapes and to display them as real-time markers. Other markers may also be shown by the imaging system, including makers displayed on the image that indicate the radial orientation of the device, structures in the tissue, etc. For example, markers may be overlaid on the image to achieve a similar result to physical markers on the catheter (e.g., electrically, magnetically, or in software). In some variations, a marker can be aligned with a distinguishing feature of the inserted device (e.g., catheter), such as a fixed jog or exposed cutter, to aid in steering or cutting with the device.

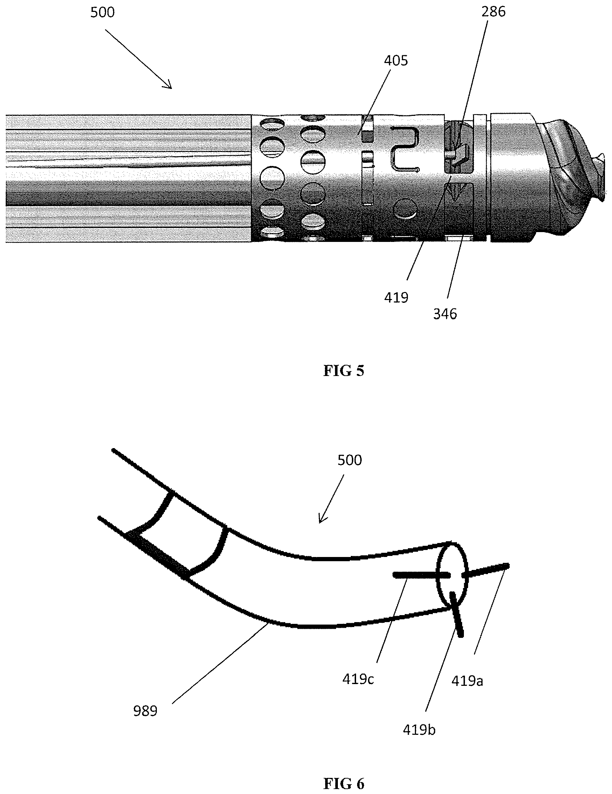

For example, as shown in FIG. 5, an occlusion-crossing device 500 can include a chassis 405 having three window regions 346 separated by spines 419 (which may be referred to as posts, struts, dividers, separators, etc.) arranged annularly around the chassis 405. These spines 419 may serve as reference markers as the imaging sensor 286 rotates and views the tissue through the windows 346. For example, spines may produce reference regions 744a, 744b, and 744c such as those shown in FIGS. 1-3. In some embodiments, as shown in FIG. 6, the spines 419 can be aligned relative to a jog 989 in the device (here such that the middle spine 419b is aligned opposite to the jog direction 989). This relative orientation can assist in pointing the jog and thus the end of the device in the desired direction.

The markers can thus produce corresponding reference regions in the images. Thus, in FIGS. 1-3, reference regions 744a, 744b, 744c in the form of striped rays indicate the locations of the markers or spines 419a, 419b, 419c, respectively, on the device 500. The reference regions 744a, 744b, 744c can thus indicate the orientation of the distal end of the catheter within the body.

During a CTO procedure, one goal may be to steer the catheter towards the plaque or unhealthy tissue. Because the middle spine 419b is aligned opposite to the jog 989 (as shown in FIG. 6), the ray 744b corresponding to the middle spine 419b can be oriented opposite to the non-healthy tissue or plaque 111 (indicated by the substantially linear portion 109 between the crescent wings 101a, 101b) to steer the catheter in the correct direction. FIG. 1 shows the catheter deflected toward the layered, healthy tissue. FIG. 2 shows the catheter rotated such that it is deflected toward the unhealthy, non-layered structure.

Thus, the system may be configured to allow the orientation of the catheter to be rotated into the correct position using fixed directional markers and the characteristic crescent wing morphology of the OCT images. It is to be understood that, although the images 100, 200, 300 are described as resulting from using a device similar to the occlusion-crossing device 500 that other device designs can be steered using the same morphology (for example, devices having different reference markers).

The crescent morphology described here can also provide direct, real-time feedback during an interventional procedure for general device repositioning based on the thickness of the layered wall structures, helping prevent perforation of the vessel. For example, the crescent morphology may indicate a plaque, however if the nearby layered wall structures appear to be very thin and perivascular structures can be seen in the OCT images beyond the layered structures, it is possible that the vessel is close to being perforated. In this case, the OCT image may serve as a warning sign, and the physician may pull the device proximal to reposition for a different approach.

An imaging system, and particularly an OCT imaging system as described, may be configured to detect, label, and/or highlight the crescent-shaped morphological structures in an image, and/or use them to automatically detect or suggest the location of an atheroma. For example, an imaging system may include a controller configured to automatically identify the crescent-shaped morphology within the OCT images. In some variations, this analysis is done separately from the imaging system (e.g., either concurrent or in real-time, or later, including as a post-procedure analysis). Likewise, in some embodiments, a controller can be configured to orient or steer a device through the vessel based upon images showing the characteristic crescent-shaped morphology. Standard image-processing algorithms may be used or adapted for use to identify the characteristic pair of crescents, which typically occur from the lumen of the vessel, radiating outward into the vessel wall. An imaging and/or image-processing system may execute (e.g., as executable code, firmware, software, hardware, etc.) image analysis logic that can determine if the crescent-shaped morphological structures are present in an image and/or indicate that they are present on the image. For example, analysis logic may determine if the structures are present in an OCT image, and may also identify them in any appropriate manner, e.g., by marking, etc. Likewise, the imaging and/or image-processing system may execute image analysis logic that can determine the direction in which the crescent-shaped structures point and, thus, the location of an atheroma therebetween.

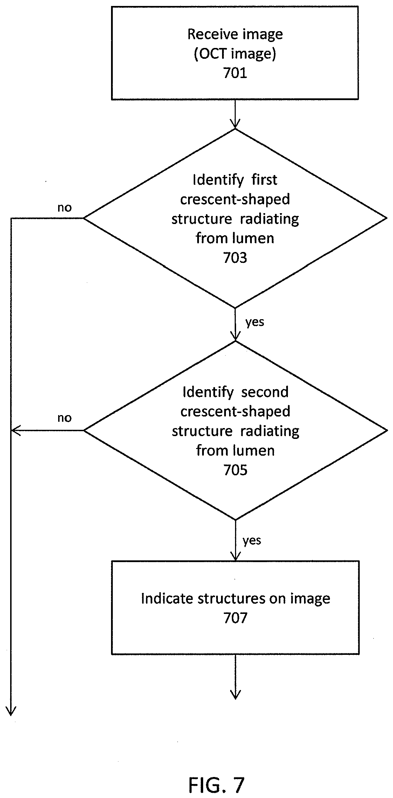

FIG. 7 shows a schematic of one variation of a method of implementing an automatic detection of the crescent-shaped structures as discussed above. At step 701, an image, such as an OCT image, from within an anatomical lumen is obtained. At step 703, it can be determined whether there is a crescent-shaped structure radiating from the lumen in the image. If so, then it can be determined at step 705 whether there is a second crescent-shaped structure radiating from the lumen in the image. If so, then, at step 707, the first and second crescent-shaped structures can be used to identify anatomical structures within the lumen, such as a plaque mass and/or healthy tissue within the lumen.

Devices or systems, including atherectomy devices and/or systems, can be designed to take advantage of the morphology resulting from the crossing of the lesion and the previously unsuspected ability to reliably determine atheroma using these newly-recognized morphological markers. For example, a device may include a pre-shaped tip region that slides easily along/between the crescent shaped morphology. Thus, a therapeutic tool may take a shape that fits into the form factor provided by this morphology, facilitating advancement through a lesion, treatment of a plaque mass, and/or delivery of a therapeutic agent (e.g., pharmaceutical).

Thus, a device can utilize the shape that is formed at the boundary between the healthy vessel and the disease when the open lumen is distended by a dissection device. The device can utilize a form factor which matches that of the dissection that is observed at the interface between the disease and the healthy vessel. The profile of the tip of the device is formed into the crescent shape described above.

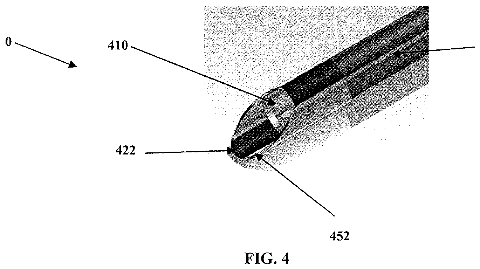

For example, referring to FIG. 4, in one variation, the distal end of an atherectomy device 400 having a rotating cutter may be adapted to take advantage of the characteristic morphology of the vessel. As shown in FIG. 4, the atherectomy device includes an elongate catheter body 401 and a rotatable cutter 410 near the distal end of the catheter body 401. The elongate body ends in a beveled tip 422 (i.e. with a troweled or tongue-shaped opening) that extends distally past the rotatable cutter 410 on one side of the cutter 410 and proximal of the rotatable cutter 410 on the other side of the cutter 410. The troweled or tongue-shaped tip 422 is thus configured to protect a portion of the rotatable cutter 410 while the exposed portion of the cutter 410 is configured to be placed in the cleft of the crescent. The troweled or tongue-shaped tip 422 has an outer curved surface configured to slide along the healthy tissue of the vessel wall and a scooped inner edge 452 configured to at least partially plane along the lesion. Thus, referring to FIGS. 1-3, the outer curved surface is configured to be placed against the rounded portion 108 and the proximal end of the scooped inner edge 452 is configured to plane between the cat ears 101a, 101b. Such a configuration would thus place the cutter 410 against the substantially linear portion 109.

The cutter 410 is actuated by a hollow drive cable which also acts to capture and store the material that is excised from the interior of the vessel. The drive cable resides in the lumen of the catheter body. An optical fiber runs through the drive cable. The distal end of the optical fiber is mounted on the cutter. The optical fiber can thus run from the cutter to a proximal connector of the device. The proximal connector provides a way to both optically and mechanically couple the fiber and drive cable to the system driving the device. The optical fiber can provide a way to generate an image of the cross-section of the vessel via OCT.

The device 400 can be easily redirected based on features apparent in the OCT image, such as this crescent wing morphology, by promoting a blunt dissection along which the tip 422 of the device 400 will track. The orientation of the device can be adjusted via visual cues generated by the OCT, such as the crescent wings. The final orientation of the device can be defined by the conformance of the shape of the tip 422 of the device 400 with the dissection plane, as described above. Advancing the device 400 pushes the cutter 410 into the linear section visible in the OCT image that is indicative of diseased tissue. In doing so, the disease region may be excised and forced into the hollow center of the drive cable. Further advancing of the device 400 pushes more disease into the cutter 410. Continuing the distal movement of the device to the distal point of the stenosis creates a patent lumen facilitating blood flow and the passage of a wire or other adjunct device past the disease. Removal of the drive cable/cutter assembly from the center of the device during the procedure would facilitate using the device sheath as an exchange or delivery catheter.

The device described herein has several advantages. For example, the device facilitates atherectomy in the coronary vasculature via image-guided cutting. Moreover, it offers a safe way to perform the procedure by orienting the device such that the tip 422 protects the healthy vessel wall from damage by the cutter.

In addition to a device having a pre-shaped tip configured to conform to the crescent-shaped tissue morphology described above, in some embodiments a device can be configured to self-orient through the vessel. In other words, the outer curved surface could automatically align with the healthy, stretched outer tissue layers (e.g., adventitia) while the beveled edge and thus the cutter could automatically align with the occlusion. In some embodiments not in accordance with the present claimed invention, therefore, the device may not require an imaging sensor.

Additional details pertinent to the present invention, including materials and manufacturing techniques, may be employed as within the level of those with skill in the relevant art. The same may hold true with respect to methods described herein in terms of additional acts commonly or logically employed. Likewise, reference to a singular item, includes the possibility that there are plural of the same items present. More specifically, as used herein and in the appended claims, the singular forms "a," "and," "said," and "the" include plural referents unless the context clearly dictates otherwise. Unless defined otherwise herein, all technical and scientific terms used herein have the same meaning as commonly understood by one of ordinary skill in the art to which this invention belongs. The breadth of the present invention is not to be limited by the examples described herein, but only by the plain meaning of the claim terms employed.

## Claims

1. An atherectomy device (400) having a distal end configured to dissect plaque from within a vessel, the device comprising:
an elongate catheter body (401);
a troweled distal tip (422) extending from the catheter body, the troweled distal tip having a curved outer surface configured to conform to the vessel and a scooped inner edge (452) configured to at least partially plane along the plaque, wherein a profile of the distal tip is in a crescent shape;
a rotatable cutter (410) at least partially within the troweled distal tip;
a hollow drive cable within a lumen of the catheter body configured to actuate the cutter; and
an optical fiber running through the drive cable, a distal end of the optical fiber mounted on the rotatable cutter, wherein the optical fiber is configured to generate an image of a cross-section of the vessel through optical coherence tomography.

2. The atherectomy device of claim 1, wherein the optical fiber is configured to image radially into the vessel.

3. The atherectomy device of claim 1, further comprising an inner lumen opening through the rotatable cutter (410) into which material cut by the device may be driven.

4. The atherectomy catheter of claim 1, wherein the rotatable cutter (410) is partially covered by the curved outer surface and is partially exposed proximate to the scooped inner edge (452).

5. The atherectomy device of claim 1, wherein the device is configured to self-orient within the vessel.

## Patentansprüche

1. Atherektomievorrichtung (400) mit einem distalen Ende, das konfiguriert ist, um Plaque aus dem Inneren eines Gefäßes zu zerlegen, wobei die Vorrichtung Folgendes umfasst:
einen langgestreckten Katheterkörper (401);
eine gewölbte distale Spitze (422), die sich vom Katheterkörper erstreckt, wobei die gewölbte distale Spitze eine gekrümmte Außenfläche, die konfiguriert ist, um sich dem Gefäß anzupassen, und eine geschaufelte Innenkante (452), die konfiguriert ist, um zumindest teilweise entlang der Plaque zu gleiten, aufweist, wobei ein Profil der distalen Spitze sichelförmig ist;
einen drehbaren Fräser (410), der zumindest teilweise in der gewölbten distalen Spitze angeordnet ist;
ein hohles Antriebskabel in einem Lumen des Katheterkörpers, das konfiguriert ist, um den Fräser zu betätigen; und
eine optische Faser, die durch das Antriebskabel verläuft, wobei ein distales Ende der optischen Faser auf dem drehbaren Fräser montiert ist, wobei die optische Faser konfiguriert ist, um ein Bild einer Schnittansicht des Gefäßes durch optische Kohärenztomografie zu erzeugen.

2. Atherektomievorrichtung nach Anspruch 1, wobei die optische Faser konfiguriert ist, um radial das Innere des Gefäßes abzubilden.

3. Atherektomievorrichtung nach Anspruch 1, ferner umfassend eine innere Lumenöffnung durch den drehbaren Fräser (410), in die von der Vorrichtung geschnittenes Material eingebracht werden kann.

4. Atherektomiekatheter nach Anspruch 1, wobei der drehbare Fräser (410) teilweise von der gekrümmten Außenfläche bedeckt und teilweise in der Nähe der geschaufelten Innenkante (452) freigelegt ist.

5. Atherektomievorrichtung nach Anspruch 1, wobei die Vorrichtung konfiguriert ist, um sich im Gefäß selbst auszurichten.

## Revendications

1. Dispositif d'athérectomie (400) ayant une extrémité distale configurée pour disséquer la plaque depuis l'intérieur d'un vaisseau, le dispositif comprenant :
un corps de cathéter allongé (401) ;
une pointe distale striée (422) s'étendant depuis le corps de cathéter, la pointe distale striée présentant une surface extérieure incurvée configurée pour s'adapter au vaisseau et un bord intérieur évidé (452) configuré pour s'aplatir, au moins partiellement, le long de la plaque, un profil de la pointe distale étant en forme de croissant ;
une lame rotative (410) au moins partiellement à l'intérieur de la pointe distale striée ;
un câble de commande creux dans une lumière du corps de cathéter, configuré pour actionner la lame ; et
une fibre optique s'étendant dans le câble de commande, une extrémité distale de la fibre optique étant montée sur la lame rotative, la fibre optique étant configurée pour générer l'image d'une section transversale du vaisseau par tomographie à cohérence optique.

2. Dispositif d'athérectomie selon la revendication 1, dans lequel la fibre optique est configurée pour réaliser une image radiale du vaisseau.

3. Dispositif d'athérectomie selon la revendication 1, comprenant en outre une lumière interne s'ouvrant à travers la lame rotative (410), dans laquelle un matériau coupé par le dispositif peut être entraîné.

4. Cathéter d'athérectomie selon la revendication 1, dans lequel la lame rotative (410) est partiellement recouverte par la surface extérieure incurvée, et est partiellement exposée à proximité du bord intérieur évidé (452).

5. Dispositif d'athérectomie selon la revendication 1, dans lequel le dispositif est configuré pour s'auto-orienter à l'intérieur du vaisseau.
